# EUROPEAN PATENT APPLICATION

(11) **EP 4 438 742 A1**
(43) Date of publication of application: **02.10.2024**
(21) Application number: 22901627.4
(22) Date of filing: 16.11.2022
(51) Int. Cl.: C12Q 1/6883, G01N 33/68

(54) **USE OF SET7/9 FOR DIAGNOSING AND TREATING ENDMT-RELATED DISEASE**

(30) Priority: 01.12.2021 KR 20210170147; 14.11.2022 KR 20220151892
(71) Applicant: IBMsol Co., Ltd., Seoul 02447 (KR)
(72) Inventor: KIM, Jongmin, Uiwang-si Gyeonggi-do 16025 (KR); KOOK, Yunjin, Seoul 06995 (KR); LEE, Aram, Seoul 02721 (KR); YUN, Eunsik, Seoul 04307 (KR)
(74) Representative: BCKIP Part mbB
(86) International application number: PCT/KR2022/018093
(87) International publication number: WO 2023/101269

(57) **Abstract**

The present invention relates to a use of SET7/9 for diagnosing and treating an endothelial-mesenchymal transition (EndMT)-related disease, and the present inventors have confirmed that a simultaneous treatment of TGF-β2 and IL-1β is a strong inducing condition for endothelial-mesenchymal transition, methyltransferase SET7/9 is an important epigenetic factor in a process of inducing endothelial-mesenchymal transition, and SET7/9 inhibition regulates pro-fibrotic signaling and inhibits endothelial-mesenchymal transition. In addition, it was found that a vascular endothelial cell dysfunction caused by endothelial-mesenchymal transition was restored by the SET7/9 inhibition and the endothelial-mesenchymal transition was closely related to a pulmonary vascular disease.

## Description

### [Technical Field]

The present invention relates to a use of SET7/9 for diagnosing and treating an endothelial-mesenchymal transition (EndMT)-related disease.

### [Background Art]

Endothelial-mesenchymal transition (EndMT) is a general term for a process in which the essence of vascular endothelial cells disappears due to various external stimuli and the vascular endothelial cells transform into cells showing mesenchymal cell characteristics that may be redifferentiated into cells such as mesenchymal stem cells, smooth muscle cells, and fibroblasts.

Until now, according to studies related to the EndMT, there are reports that fibrosis of the endothelial cells causes or worsens a series of diseases such as cancer or arteriosclerosis along with inflammatory responses, and regarding various external stimuli that cause damage to the endothelial cells, many related studies are still in progress.

Meanwhile, conventional EndMT is known to cause the formation of fibroblasts and myofibroblasts that pathologically affect cancer and fibrosis. According to a recent new study, it is known that transformed cells may differentiate into chondroblasts, osteoblasts, and adipoblasts, respectively. However, studies on EndMT are significantly inadequate compared to studies on epithelial-mesenchymal transition (EMT).

Currently, institutions and companies to which research developers on related diseases caused by EndMT belong are investing heavily in related techniques, and are making efforts to improve the productivity of these investments. A typical work process of screening includes compound dispensing, dilution, mixing of screening components, culturing and detection, analysis of screening data, and reporting of results, and data is secured by screening millions of libraries using such a system, but a novel screening method is continuously required to more quickly and efficiently screen libraries for thousands of active substances that are expected to be derived from detailed studies in the future.

### [Disclosure]

### [Technical Problem]

An object of the present invention is to provide a biomarker composition for diagnosing an endothelial-mesenchymal transition (EndMT)-related disease containing methyltransferase SET7/9 protein or a gene encoding methyltransferase SET7/9 protein as an active ingredient.

Another object of the present invention is to provide a composition for diagnosing an EndMT-related disease containing an agent capable of measuring an expression level of methyltransferase SET7/9 as an active ingredient, and a kit for diagnosing an EndMT-related disease containing the composition.

Still another object of the present invention is to provide a method for providing information required for diagnosing an EndMT-related disease by measuring an expression level of SET7/9 in a sample isolated from a patient.

Still another object of the present invention is to provide a method for screening a therapeutic agent for an EndMT-related disease by measuring an expression or activity level of SET7/9 in vascular endothelial cells.

Still another object of the present invention is to provide a pharmaceutical composition for preventing or treating pulmonary arterial hypertension containing a SET7/9 expression or activity inhibitor as an active ingredient.

Still another object of the present invention is to provide a reagent composition for inhibiting EndMT in pulmonary artery endothelial cells in vitro or in pulmonary arterial hypertension rat models in vivo, the reagent composition containing a SET7/9 expression or activity inhibitor as an active ingredient.

Still another object of the present invention is to provide a method for inhibiting EndMT in pulmonary artery endothelial cells or in pulmonary arterial hypertension rat models, the method including treating pulmonary artery endothelial cells in vitro or pulmonary arterial hypertension rat models in vivo with a SET7/9 expression or activity inhibitor.

### [Technical Solution]

In order to achieve the above objects, the present invention provides a biomarker composition for diagnosing an endothelial-mesenchymal transition (EndMT)-related disease containing methyltransferase SET7/9 protein or a gene encoding methyltransferase SET7/9 protein as an active ingredient.

In addition, the present invention provides a composition for diagnosing an EndMT-related disease containing an agent capable of measuring an expression level of methyltransferase SET7/9 as an active ingredient.

In addition, the present invention provides a kit for diagnosing an EndMT-related disease containing the composition for diagnosing an EndMT-related disease.

In addition, the present invention provides a method for providing information required for diagnosing an EndMT-related disease, the method including: (1) measuring an mRNA expression level of SET7/9 gene or an expression level of SET7/9 protein from a sample isolated from a patient; (2) comparing the mRNA expression level of the SET7/9 gene or the expression level of the SET7/9 protein with an expression level of a control sample; and (3) determining that a disease is an EndMT-related disease when the mRNA expression level of the SET7/9 gene or the expression level of the SET7/9 protein is higher than that of the control sample.

In addition, the present invention provides a method for screening a therapeutic agent for an EndMT-related disease, the method including: (1) bringing test substances into contact with vascular endothelial cells; (2) measuring an expression or activity level of SET7/9 protein in the vascular endothelial cells in contact with the test substances; and (3) screening a test substance having a reduced expression or activity level of the SET7/9 protein compared to a control sample.

In addition, the present invention provides a pharmaceutical composition for preventing or treating pulmonary arterial hypertension containing a SET7/9 expression or activity inhibitor as an active ingredient.

In addition, the present invention provides a reagent composition for inhibiting EndMT in pulmonary artery endothelial cells in vitro or in pulmonary arterial hypertension rat models in vivo, the reagent composition containing a SET7/9 expression or activity inhibitor as an active ingredient.

In addition, the present invention provides a method for inhibiting EndMT in pulmonary artery endothelial cells or in pulmonary arterial hypertension rat models, the method including treating pulmonary artery endothelial cells in vitro or pulmonary arterial hypertension rat models in vivo with a SET7/9 expression or activity inhibitor.

### [Advantageous Effects]

The present invention relates to a use of SET7/9 for diagnosing and treating an endothelial-mesenchymal transition (EndMT)-related disease, and the present inventors have confirmed that a simultaneous treatment of TGF-β2 and IL-1β is a strong inducing condition for endothelial-mesenchymal transition, methyltransferase SET7/9 is an important epigenetic factor in a process of inducing endothelial-mesenchymal transition, and SET7/9 inhibition regulates pro-fibrotic signaling and inhibits endothelial-mesenchymal transition. In addition, it was found that a vascular endothelial cell dysfunction caused by endothelial-mesenchymal transition was restored by the SET7/9 inhibition and the endothelial-mesenchymal transition was closely related to a pulmonary vascular disease.

### [Description of Drawings]

FIG. 1 illustrates the results showing that a simultaneous treatment of TGF-β2 and IL-1β induces strong endothelial-mesenchymal transition (EndMT) in human PAECs.
FIG. 2 illustrates the results showing that methyltransferase SET7/9 is required for changes in endothelial-mesenchymal transition gene expression induced by TGF-β2 and IL-1β.
FIG. 3 illustrates the results showing that SET7/9 regulates pro-fibrotic signaling induced by the simultaneous treatment of TGF-β2 and IL-1β.
FIG. 4 illustrates the results showing that in hPAECs, SET7/9 is mainly located in the cytoplasm and regulates TGF-βR1 expression through methylation.
FIG. 5 illustrates the results showing that inhibition of SET7/9 restores a vascular endothelial cell dysfunction induced by TGF-β2 and IL-1β.
FIG. 6 illustrates the results showing that EndMT occurs in a pulmonary vascular disease, and SET7/9 is significantly upregulated in pulmonary arterial hypertension animal models and pulmonary fibrosis animal models.
FIG. 7 illustrates the results of restoration of endothelial-mesenchymal transition by SET7/9 inhibitor cyproheptadine.
FIG. 8 illustrates the results of the effect of the SET7/9 inhibitor cyproheptadine in a SuHx-induced pulmonary arterial hypertension animal model.
FIG. 9 illustrates the results of restoration of endothelial-mesenchymal transition by SET7/9 specific inhibitor (R)-PFI-2.
FIG. 10 illustrates the results of the effect of the SET7/9 specific inhibitor (R)-PFI-2 in a SuHx-induced pulmonary arterial hypertension animal model.

### [Best Mode for Invention]

The present invention provides a biomarker composition for diagnosing an endothelial-mesenchymal transition (EndMT)-related disease containing methyltransferase SET7/9 protein or a gene encoding methyltransferase SET7/9 protein as an active ingredient.

Preferably, the EndMT-related disease may be pulmonary fibrosis or pulmonary arterial hypertension, but is not limited thereto.

In addition, the present invention provides a composition for diagnosing an EndMT-related disease containing an agent capable of measuring an expression level of methyltransferase SET7/9 as an active ingredient.

Specifically, the agent capable of measuring an expression level of SET7/9 may be a primer or probe that specifically binds to the SET7/9 gene, or an antibody, peptide, aptamer, or compound that specifically binds to the SET7/9 protein, but is not limited thereto.

Preferably, the EndMT-related disease may be pulmonary fibrosis or pulmonary arterial hypertension, but is not limited thereto.

In addition, the present invention provides a kit for diagnosing an EndMT-related disease containing the composition for diagnosing an EndMT-related disease.

Meanwhile, the kit of the present invention may contain an antibody that specifically binds to a marker component, a secondary antibody conjugate conjugated with a label that develops color by a reaction with a substrate, a chromogenic substrate solution that undergoes a color reaction with the label, a cleaning solution, an enzyme reaction stopping solution, and the like, and may be produced in a number of separate packaging and compartments containing reagent components to be used.

In the present specification, the term "diagnosis" includes determining susceptibility of a subject to a particular disease or disorder, determining whether a subject currently has a particular disease or disorder; determining a prognosis of a subject suffering from a specific disease or disorder, or therametrics (for example, monitoring conditions of a subject to provide information on treatment efficacy).

In the present specification, the term "primer" refers to a short nucleic acid sequence having a short free 3'-hydroxyl group, which may form base pairs with a complementary template and acts as a starting point for template strand replication. The primer may initiate DNA synthesis in the presence of a reagent for polymerization (that is, DNA polymerase or reverse transcriptase) under an appropriate buffer solution and temperature and four different nucleoside triphosphates. The PCR conditions and the lengths of the sense and antisense primers may be appropriately selected according to a technique known in the art.

In the present specification, the term "probe" refers to a nucleic acid fragment such as RNA or DNA corresponding to a few bases or several hundreds of bases that may specifically bind to mRNA, and is labeled so that the presence or absence and an expression level of a specific mRNA may be confirmed. The probe may be prepared in the form of an oligonucleotide probe, a single strand DNA probe, a double strand DNA probe, or an RNA probe. The selection of suitable probes and hybridization conditions may be appropriately selected according to a technique known in the art.

In the present specification, the term "antibody" is well known in the art and refers to a specific immunoglobulin directed against an antigenic site. The antibody in the present invention refers to an antibody that specifically binds to SET7/9 of the present invention, and may be prepared according to a conventional method in the art. The form of the antibody includes a polyclonal antibody or a monoclonal antibody, and includes all immunoglobulin antibodies. The antibody refers to a complete form having two full-length light chains and two full-length heavy chains. In addition, the antibody also includes a special antibody such as a humanized antibody.

In the present specification, the term "peptide" refers to a compound that has a high binding strength to a target substance and does not cause denaturation during a thermal/chemical treatment. In addition, the peptide may be attached to another protein and used as a fusion protein because its molecular size is small. Specifically, the peptide may be used as a diagnostic kit and a drug delivery material because the peptide may be attached to a polymer protein chain.

In the present specification, the term "aptamer" refers to a polynucleotide composed of a specific type of single-stranded nucleic acid (DNA, RNA, or modified nucleic acid) having a stable tertiary structure by itself and having a property capable of binding to a target molecule with high affinity and specificity. As described above, since the aptamer is composed of a polynucleotide that has higher stability than a protein, has a simple structure, and is easily synthesized, while being capable of specifically binding to an antigenic material like an antibody, the aptamer may be used instead of an antibody.

In addition, the present invention provides a method for providing information required for diagnosing an EndMT-related disease, the method including: (1) measuring an mRNA expression level of SET7/9 gene or an expression level of SET7/9 protein from a sample isolated from a patient; (2) comparing the mRNA expression level of the SET7/9 gene or the expression level of the SET7/9 protein with an expression level of a control sample; and (3) determining that a disease is an EndMT-related disease when the mRNA expression level of the SET7/9 gene or the expression level of the SET7/9 protein is higher than that of the control sample.

Preferably, the EndMT-related disease may be pulmonary fibrosis or pulmonary arterial hypertension, but is not limited thereto.

In the present specification, the term "sample isolated from a patient" includes a sample such as a tissue, cell, whole blood, serum, plasma, saliva, sputum, cerebrospinal fluid, or urine that differs from a control in the expression level of the SET7/9 gene or SET7/9 protein, but is not limited thereto.

Specifically, as a method for measuring the mRNA expression level, RT-PCR, competitive RT-PCR, real-time RT-PCR, RNase protection assay, Northern blotting, and DNA chips are used, but the present invention is not limited thereto.

Specifically, as a method for measuring the protein expression level, Western blot, enzyme linked immunosorbent assay (ELISA), Radioimmunoassay (RIA), radioimmunodiffusion, Ouchterlony immunodiffusion, rocket immunoelectrophoresis, tissue immunostaining, immunoprecipitation assay, complement fixation assay, FACS, and protein chips are used, but the present invention is not limited thereto.

In addition, the present invention provides a method for screening a therapeutic agent for an EndMT-related disease, the method including: (1) bringing test substances into contact with vascular endothelial cells; (2) measuring an expression or activity level of SET7/9 protein in the vascular endothelial cells in contact with the test substances; and (3) screening a test substance having a reduced expression or activity level of the SET7/9 protein compared to a control sample.

The term "test substance" used while referring to the screening method of the present invention refers to an unknown candidate substance used in screening to test whether the test substance affects an expression level of a gene or affects expression or activity of a protein. The sample includes a chemical substance, a nucleotide, antisense-RNA, small interference RNA (siRNA), and a natural extract, but is not limited thereto.

In addition, the present invention provides a pharmaceutical composition for preventing or treating pulmonary arterial hypertension containing a SET7/9 expression or activity inhibitor as an active ingredient.

Preferably, the SET7/9 expression or activity inhibitor may be an antisense nucleotide, small interfering RNA (siRNA), short hairpin RNA (shRNA), a compound, a peptide, an aptamer, and an antibody that specifically bind to SET7/9, and more preferably, the SET7/9 expression or activity inhibitor may be (R)-PFI-2, but the present invention is not limited thereto.

The pharmaceutical composition of the present invention may be prepared using a pharmaceutically acceptable and physiologically acceptable adjuvant in addition to the active ingredient, and such as an excipient, a disintegrant, a sweetener, a binder, a coating agent, an expander, a lubricant, a slip modifier, a flavoring agent or a solubilizer may be used as the adjuvant. The pharmaceutical composition of the present invention may be preferably formulated into a pharmaceutical composition containing one or more pharmaceutically acceptable carriers in addition to the active ingredient for administration. In the composition formulated into a liquid solution, a pharmaceutically acceptable carrier may be sterile and biocompatible and used by mixing saline, sterile water, Ringer's solution, buffered saline, albumin injection, dextrose solution, maltodextrin solution, glycerol, ethanol, and one or more of these ingredients, and if necessary, other common additives such as an antioxidant, a buffer, and a bacteriostatic agent may be added. In addition, a diluent, a dispersant, a surfactant, a binder, and a lubricant may be additionally added to formulate the pharmaceutical composition into a formulation for injection such as an aqueous solution, a suspension, and an emulsion, pills, capsules, granules, or tablets.

The pharmaceutical formulation of the pharmaceutical composition of the present invention may be granules, powder, coated tablets, tablets, capsules, suppositories, syrups, juices, suspensions, emulsions, drops or injectable solutions, and sustained-release preparations of an active compound. The pharmaceutical composition of the present invention may be administered in a common manner via an intravenous, intramuscular, intraarterial, intraperitoneal, intrasternal, transdermal, intranasal, inhalational, topical, rectal, oral, intraocular, or intradermal route. An effective amount of the active ingredient of the pharmaceutical composition of the present invention refers to an amount required for preventing or treating a disease. Therefore, the effective amount may be controlled by various factors including the type of disease, the severity of the disease, the types and contents of the active ingredient and other ingredients contained in the composition, the type of formulation, an age, weight, general health conditions, gender, and diet of a patient, an administration time, an administration route, a secretion rate of the composition, a treatment period, and drugs used in combination. Although not limited thereto, for example, in the case of adults, when administered once or several times, the composition of the present invention may be administered at a dosage of 0.1 ng/kg to 10 g/kg when the composition is a compound, at a dosage of 0.1 ng/kg to 10 g/kg when the composition is a polypeptide, protein, or antibody, and at a dosage of 0.01 ng/kg to 10 g/kg when the composition is an antisense nucleotide, siRNA, shRNAi, or miRNA.

In addition, the present invention provides a reagent composition for inhibiting EndMT in pulmonary artery endothelial cells in vitro or in pulmonary arterial hypertension rat models in vivo, the reagent composition containing a SET7/9 expression or activity inhibitor as an active ingredient.

Preferably, the SET7/9 expression or activity inhibitor may be cyproheptadine or (R)-PFI-2, but is not limited thereto.

In addition, the present invention provides a method for inhibiting EndMT in pulmonary artery endothelial cells or in pulmonary arterial hypertension rat models, the method including treating pulmonary artery endothelial cells in vitro or pulmonary arterial hypertension rat models in vivo with a SET7/9 expression or activity inhibitor.

Preferably, the SET7/9 expression or activity inhibitor may be cyproheptadine or (R)-PFI-2, but is not limited thereto.

### [Mode for Invention]

Hereinafter, the present invention will be described in detail with reference to examples to help the understanding of the present invention. However, the following examples illustrate only the present invention, and the scope of the present invention is not limited by the following examples. The examples of the present invention are provided in order to completely explain the present invention to those skilled in the art.

### <Experimental Examples>

The following experimental examples are intended to provide experimental examples commonly applied to each example according to the present invention.

### 1. Experimental Animals

In the bleomycin-induced PF mouse models, 8-week-old C57BL/6 mice (specific-pathogen-free grade from SaeronBio, Republic of Korea) were used. When the mice were under respiratory anesthesia, 2 U/kg of bleomycin (Sigma, B2434) dissolved in 1 × sterile phosphate-buffered saline (PBS) was injected intratracheally. The lungs of the mice were harvested 3 weeks after the bleomycin treatment.

8-Week-old male Sprague Dawley rats (specific-pathogen-free grade form SaeronBio, Republic of Korea) were used for the PAH rat model.

Semaxanib (DC Chemicals, SU5416) dissolved in DMSO and then suspended in a buffer (0.5% sodium carboxymethylcellulose, 0.5% sodium chloride, 0.4% Tween-80, and 0.9% benzyl alcohol that were dissolved in deionized water) was injected subcutaneously into the rats at 20 mg/kg. The rats were exposed to 10% O₂ hypoxic conditions for 3 weeks. After the rats were restored to normoxic conditions, Cyproheptadine (Cayman) or (R)-PFI-2 (DC Chemicals) was administered. Cyproheptadine was administered intraperitoneally every day at 5 mg/kg for 2 weeks, and (R)-PFI-2 was administered subcutaneously every 2 days at 10 mg/kg for 3 weeks. A right ventricular systolic pressure (RVSP) was measured, and the rats were sacrificed.

### 2. Cell Culture and Transfection

Pulmonary arterial ECs (PAECs) and human umbilical vein ECs (HUVECs) were cultured in EGM-2 (Lonza) to which MycoZap (Lonza) and 1% penicillin-streptomycin (Welgene) were added. Human Microvascular ECs (HMVECs) were cultured in EGM-2-MV (Lonza), and Lenti-X-293T and MCF7 cells were cultured in Dulbecco's modified Eagle medium (DMEM) (HyClone) containing 10% fetal bovine serum (FBS, HyClone), 1% penicillin-streptomycin (Welgene), and MycoZap (Lonza) . All cell lines were cultured at 37°C in a 5% CO₂ incubator and cultured at 90% density.

### 3. Reagent and Drug Treatment

TGF-β2 was purchased from Peprotech and dissolved in pure water containing 0.1% bovine serum albumin (BSA) . IL-1β was purchased from R&D System and dissolved in 1 × PBS containing 0.1% BSA. TGF-β2 (10ng/ml) and IL-1β (1ng/ml) were used at a specific time point. PAECs were washed twice with 1 × PBS and then treated with the drugs together with EGM-2 (Lonza) to which 10% FBS, 1% penicillin-streptomycin, and MycoZap were added.

### 4. RNA Extraction and Quantitative Real-Time PCR

In order to isolate the total RNA, miRNeasy RNA isolation kit (QIAGEN) was used. The purified RNA was reverse transcribed using qPCRBIO cDNA Synthesis Kit (PCR-BIOSYSTEMS), and real-time quantitative PCR (qRT-PCR) was performed using qPCRBIO SyGreen Mix Hi-ROX (PCR-BIOSYSTEMS) according to the manufacturer's instructions. The RNA extract from animal lung tissue was homogenized at a frequency of 25 Hz for 3 minutes and dissolved in QIAZOL (provided by the miRNeasy RNA isolation kit). In order to measure the relative expression of RNA, ribosomal 18S RNA was used as an internal control in vitro and in vivo. The PCR primer sequences used in qRT-PCR are listed in Table 1.

**[Table 1]**

| **Species** | **Gene name** | **Primers** |
|---|---|---|
| Human | 18s rRNA | Forward : 5'-ACCCGTTGAACCCCATTCGTGA-3' |
| | | Reverse : 5'-GCCTCACTAAACCATCCAATCGG-3' |
| | PECAM-1 | Forward : 5'-AAGTGGAGTCCAGCCGCATATC-3' |
| | | Reverse : 5'-ATGGAGCAGGACAGGTTCAGTC-3' |
| | GH5 | Forward : 5'-GAAGCCTCTGATTGGCACAGTG-3' |
| | | Reverse : 5'TTTTGTGACTCGGAAGAACTGGC-3' |
| | FN | Forward : 5'-ACAACACCGAGGTGACTGAGAC-3' |
| | | Reverse: 5'-GGACACAACGATGCTTCCTGAG-3' |
| | CDH2 | Forward : 5'-CCTCCAGAGTTTACTGCCATGAC-3' |
| | | Reverse : 3-GTAGGATCTCCGCCACTGATTC-3' |
| | ACTA2 | Forward : 5'-CTATGCCTCTGGACGCACAACT-3' |
| | | Reverse : 5'-CAGATCCAGACGCATGATGGCA-3' |
| | TAGLN | Forward : 5'-TCCAGGTCTGGCTGAAGAATGG-3' |
| | | Reverse : 5'-CTGCTCCATCTGCTTGAAGACC-3' |
| | MMP2 | Forward : 5'AGCGAGTGGATGCCGCCTTTAA-3' |
| | | Reverse : 5'-CATTCCAGGCATCTGCGATGAG-3' |
| | SNAIL | Forward : 5'-TGCCCTCAAGATGCACATCCGA-3' |
| | | Reverse : 5'-GGGACAGGAGAAGGGCTTCTC-3' |
| | SET7/9 | Forward : 5'-CGTATGTAGACGGAGAOCTGAAC-3' |
| | | Reverse : 5'-CTCCTACAAGGCTTCCTCCATC-3' |
| | COL3A1 | Forward : 5'-TGGTCTGCAAGGAATGCCTGGA-3' |
| | | Reverse: 5'-TCTTTCCCTGGGACACCATCAG-3' |
| Mouse | 18s rRNA | Forward : 5'-TTGATTAAGTCCCTGCCCTTTGT-3' |
| | | Reverse: 5'-CGATCCGAGGGCCTCACTA-3' |
| | FN | Forward : 5'-CCCTATCTCTGATACCGTTGTCC-3' |
| | | Reverse : 5'-TGCCGCAACTACTGTGATTCGG-3' |
| | CDH2 | Forward : 5'-CCTCCAGAGTTTACTGCCATGAC-3' |
| | | Reverse : 5'-CCACCACTGATTCTGTATGCCG-3' |
| | TAGLN | Forward : 5'-GCAGATGGAACAGGTGGCTCAA-3' |
| | | Reverse : 5'-CCCAAAGCCATTAGAGTCCTCTG-3' |
| | VIM | Forward : 5'-CGGAAAGTGGAATCCTTGCA-3' |
| | | Reverse: 5'-CACATCGATCTGGACATGCTGT-3' |
| | FSPI | Froward : 5-AGCTCAAGGAGCTACTGACCAG-3' |
| | | Reverse : 5'-GCTGTCCAAGTTGCTCATCACC-3' |
| | COL1A1 | Forward : 5'-CCTCAGGGTATTGCTGGACAAC-3' |
| | | Reverse: 5'-CAGAAGGACCTTGTTTGCCAGG-3' |
| | COL3A1 | Forward : 5'-GACCAAAAGGTGATGCTGGACAG-3' |
| | | Reverse : 5'-CAAGACCTCGTGCTCCAGTTAG-3' |
| | SET7/9 | Forward : 5'-TTGACGGAGAGATGCTCGAAGG-3' |
| | | Reverse : 5'-GAAGGAGAGCATCGCTGGAGAT-3' |

### 5. Immunoprecipitation and Western Blotting

All cells were lysed in radioimmunoprecipitation assay (RIPA) buffer (Biosesang) containing protease (GenDepot) and phosphatase (Sigma Aldrich) inhibitors for 20 minutes. Thereafter, the cells were centrifuged at 4°C and 13,000 rpm for 15 minutes. A protein concentration was measured using Pierce BCA Protein Assay Kit (Thermo Scientific), and Laemmli SDS Sample Buffer was added to an equal amount of protein and boiled. For immunoprecipitation, the cells were dissolved in an immunoprecipitation buffer (50 mM Tris-HCl (pH 7.4), 50 mM NaCl, 1% Triton X-100, 0.5% NP-40) containing protease (GenDepot) and phosphatase (Sigma Aldrich) for 20 minutes and then centrifuged at 4°C and 13,000 rpm for 15 minutes. Agarose beads A/G (Santa Cruz) were added to the cell lysate, pre-cleared at 4°C for 1 hour, and then reacted with antibodies at 4°C overnight. Thereafter, 20 ul of agarose beads A/G were added and then reacted at 4°C for 2 hours. Pellets formed by centrifugation of the resulting cell lysate were washed several times with an immunoprecipitation buffer, and Laemmli SDS Sample Buffer was added and boiled.

Proteins were separated by sodium dodecyl sulfate polyacrylamide gel electrophoresis (SDS-PAGE) and transferred to a polyvinylidene difluoride membrane (Millipore). After blocking the membrane with 3% BSA dissolved in 0.2% PBS with Tween^{®} detergent (PBST), the proteins on the membrane were reacted with the primary antibodies listed in Table 2 overnight. For immunodetection, HRP-conjugated secondary antibodies (1:4,000) were conjugated with the primary antibodies at room temperature for 1 hour (average temperature of 22°C), and then detected using an enhanced chemiluminescence detection system (Thermo Scientific). In order to separate cytoplasmic and nuclear proteins, EpiQuik Nuclear Extraction Kit (EpiGentek) was used according to the manufacturer's instructions. Protein extracts from animal lung tissue were homogenized in RIPA buffer for 3 minutes at a frequency of 25 Hz.

**[Table 2]**

| **Protein name** | **Dilution** |
|---|---|
| GAPDH (Cell Signaling, 2118) | 1:6,000 |
| Lamin B (Santa Cruz, sc-374015) | 1:500 |
| SET7/9 (Cell Signaling, 2813) | 1:1,000 |
| CD31 (Cell Signaling, 3528S) | 1:2,000 |
| VE-cadherin (Cell Signaling, 2500S) | 1:2,000 |
| Fibronectin (BD Pharmighen, 610077) | 1:2,000 |
| N-cadherin (BD Pharmigen, 610920) | 1:2,000 |
| α-SMA (Abcam, ab5694) | 1:4,000 |
| SM22α (Abcam, ab14106) | 1:20,000 |
| SNAIL (Cell Signaling, 3879S) | 1:1,000 |
| MMP2 (Cell Signaling, 40994) | 1:2,000 |
| TGF-βR1 (Santa Cruz, sc-398, sc-518018) | 1:1,000 |
| Slug (Cell Signaling. 9585S) | 1:1,000 |
| Collagen 1 (Novus, NB600-408) | 1:1,000 |
| Twist (Santa Cruz, sc-81417) | 1:1,000 |
| Tenascin-C (Santa Cruz, sc-20932) | 1:1,000 |
| Calponin(Abcam, ab46794) | 1:1,000 |
| Phospho-vimentin (Abcam, Ab22651) | 1:3,000 |
| Vimentin (Santa Cruz, SC-6260) | 1:2,000 |
| ZEB2 (Santa Cruz, sc-271984) | 1:1,000 |
| SMAD2 (Cell Signaling, 5339S) | 1:1,000 |
| SMAD3 (Cell Signaling, 9523S) | 1:1,000 |
| Phospho-SMAD2 (Cell Signaling, 3108) | 1:1,000 |
| Phospho-SMAD3 (Cell Signaling, 9S20S) | 1:1,000 |
| NF-κB p65 (Cell Signaling, 8242S) | 1:1,000 |
| IκBα (Cell Signaling, 9242S) | 1:1,000 |
| Phospho-IκBα (Cell Signaling, 2859S) | 1:500 |
| HA High Affinity from rat IgG1 (Roche 11867423001) | 1:2,000 |
| Methylated Lysine (di methyl, mono methyl) (Abcam, ab23366) | 1:2,000 |

### 6. Lentivirus Production and Infection

For SET7/9 overexpression in PAECs, a SET7/9 overexpression lentiviral vector (provided by Dr. Geunil Kim, Sookmyung Women's University) was used. Lenti-X-293T was used to produce lentivirus, which was transfected with a lentiviral vector along with a second-generation packaging system (psPAX2 and pCL-VSVG) using a polyethylenimine (PEI) transfection reagent. 48 hours after the transfection of the cells, a medium containing lentivirus was harvested and replaced with a fresh complete medium for an additional 24 hours. The harvested lentivirus was concentrated using Amicon Centrifugal Filter Unit (Milipore) and quantified using qPCR Lentivirus Titration Kit (Abmgood, LV900). PAECs were infected with SET7/9 overexpression lentivirus (100 multiplicity of infection [MOI]) in a serum-free medium. After 24 hours, the medium was replaced with a fresh complete medium. The above process was repeated a total of two times (for a total of 96 hours of infection).

### 7. Immunocytochemistry

PAECs and Lenti-X-293T were immobilized with 4% formaldehyde and washed with 1 × PBS. Thereafter, the cells were permeabilized with 0.2% Triton X-100 and blocked with 1% BSA dissolved in 1 × PBS for 1 hour at room temperature. The cells were reacted with the following primary antibodies at 4°C overnight: anti-VE-cadherin (Cell Signaling, 2500S, 1:100), anti-Fibronectin (BD Pharmigen, 61007, 1:100), anti-TAGLN (Abeam, ab14106, 1:200), anti-α-SMA conjugated with Cy3 (Sigma Aldrich, C6198, 1:200), anti-NF-xB p65 (Cell signaling, 8242S, 1:400), anti-SET7/9 (LsBio, C337450, 1:100), and rhodamine phalloidin (Sigma Aldrich, 1:1,000). Secondary antibodies of donkey anti-goat Alexa Fluor 488 and donkey anti-goat Alexa Fluor 568 (Invitrogen, 1:400) were reacted at room temperature for 1 hour. In order to obtain immunostaining images, a confocal microscope equipped with a 60 × objective lens (Zeiss LSM-700) was used.

### 8. Immunohistochemistry

The harvested animal lung tissue was embedded in paraffin blocks and cut into 10 um sections. The paraffin sections were deparaffinized using Histoclear for 10 minutes. In order to hydrate the sections, ethanol dissolved in pure water was used according to a concentration gradient (100%, 90%, 70%, 50%, 30%, and 0%). The tissue slides were placed in Borg Decloaker RTU antigen retrieval solution (Biocare Medical) and the sections were heated using a pressure cooker to recover antigens. When the temperature of the recovery solution was lowered, the sections were washed three times with 1 × PBS for 5 minutes each, and then blocked with a blocking buffer (1% BSA and 5% goat serum dissolved in 1 × PBS) at room temperature for 30 minutes. The sections were reacted with the primary antibodies at 4°C overnight. Anti-vWF conjugated with FITS (Abeam, ab8822, 1:50), anti-α-SMA conjugated with Cy3 (Sigma Aldrich, C6198, 1:100), anti-SM22α (Abeam, ab14106, 1:130), and anti-SET7/9 (LsBio, C337450, 1:50) were used. For fluorescent staining, the sections were reacted with the secondary antibodies such as donkey anti-goat Alexa Fluor 568 (Invitrogen, 1:150 to 1:400), Goat anti-Rabbit Alexa Fluor 647 (Invitrogen, 1:150), and donkey anti-goat Alexa Fluor 568 (Invitrogen, 1:400) at room temperature for 1 hour. For color staining, after the sections were reacted with the primary antibodies overnight, the sections were reacted with Goat anti-Mouse IgG Secondary Antibody, HRP (Pierce, 1:100) for 1 hour and subjected to a color reaction using DAB staining kit (Vector, SK-4800). Hematoxylin-eosin staining was performed using staining products available from Vector. Image analysis was performed using confocal microscopes equipped with a 40× objective available from Zeiss and Leica. The co-localization effect was verified through Mander's overlap coefficient (MOC).

### 9. Cell Migration Assay

A PAEC migration assay was performed using a 12-well plate containing SPLScar Block (SPL) according to the manufacturer's instructions. The cells were cultured in EGM-2 to which 10% FBS, 1% penicillin-streptomycin, and MycoZap were added and treated with each drug. A migration assay according to the drug treatment was performed and observation was performed with an optical microscope. Termination of migration was assayed using Image J software.

### 10. Dextran Permeation Assay

PAECs were seeded at 2 × 10⁵ cells per well in a 24-well plate equipped with a cell culture inlet (SPL, 0.4 µm). After a drug treatment, the insert was transferred to a well containing a serum-free medium (SFM), the medium in the insert was replaced with SFM containing FITC-dextran (Sigma Aldrich, FD40S, 1 mg/ml). After 10 minutes and 1 hour, a part of SFM was taken out from the plate well where dextran permeation occurred, and the fluorescence was measured. The fluorescence intensity was measured on a microplate reader (excitation, 485 nm; emission, 530 nm).

### 11. Dil-Ac-LDL Uptake Assay

PAECs were seeded at 2 × 10⁵ cells per well in a 12-well plate. After a drug treatment, the cells were reacted with Dil-Ac-LDL (Invitrogen, 10 µg/ml) at 37°C for 3 hours. After the reaction, the cells were washed four times with 1 × PBS and then subjected to image analysis with a fluorescence microscope.

### 12. Statistical Analysis

All experimental data were analyzed after at least three times of experiments and verified using GraphPad Prism 5.0 and 7.0 software. Statistical differences were analyzed using an unpaired two-tailed Student's t-test to compare two groups. When there were three or more groups, analysis was performed by One-way ANOVA. p-value < 0.05 was considered statistically significant.

### <Example 1> Simultaneous Treatment of TGF-β2 and IL-1β, Which Are Strong Conditions for Inducing Endothelial-Mesenchymal Transition (EndMT)

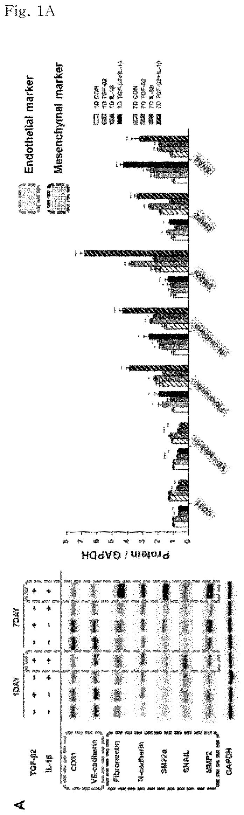

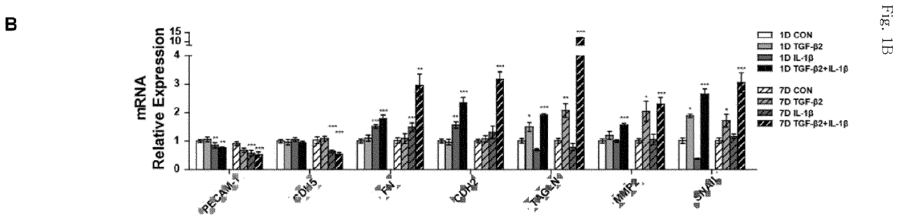

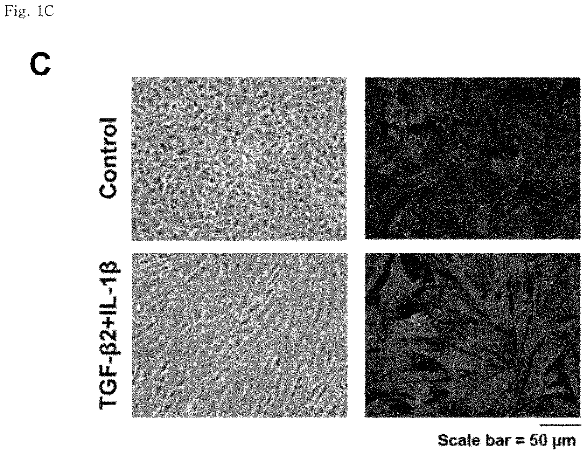

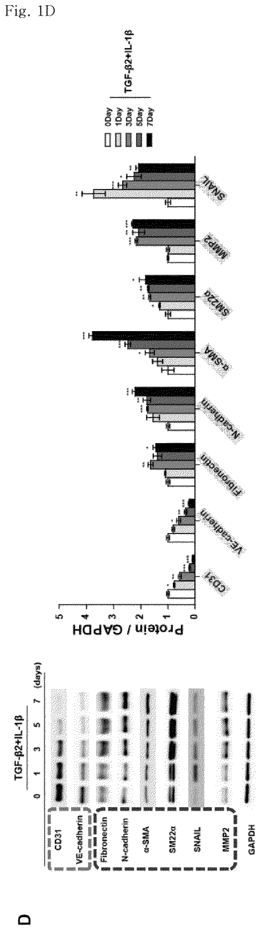

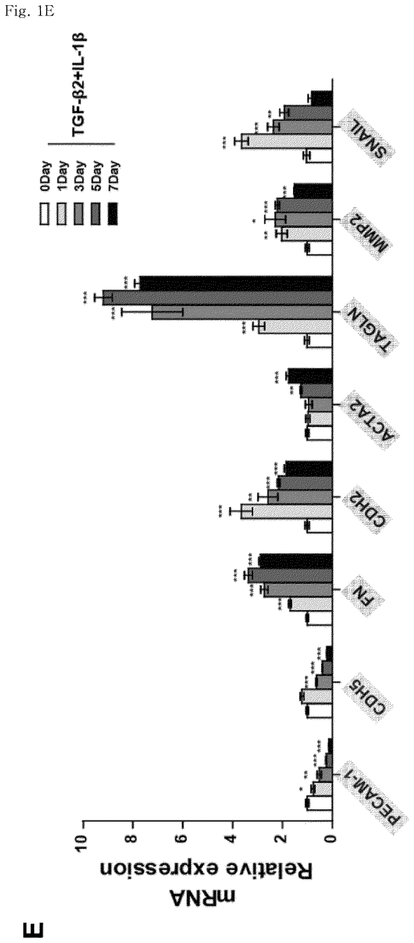

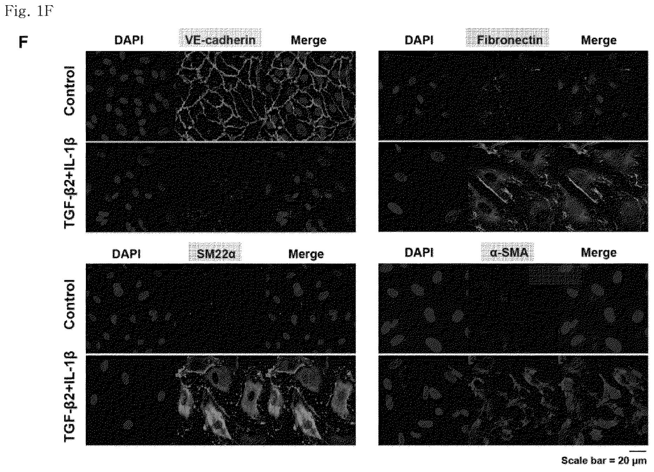

In order to determine whether a simultaneous treatment of TGF-β2 and IL-1β is a strong inducer of endothelial-mesenchymal transition, a degree of endothelial-mesenchymal transition was compared by treating the two drugs separately or simultaneously. The results of Western blot (FIG. 1A) and qRT-PCR (FIG. 1B) showed that vascular endothelial cell markers CD31 (PECAM1) and VE-cadherin (CDH5) decreased more in a group treated with both drugs simultaneously than when each drug was treated alone. Mesenchymal cell markers Fibronectin (FN), N-cadherin (CDH2), SM22α (TAGLN), MMP2, and SNAIL also increased more in the group treated with both drugs simultaneously. When endothelial-mesenchymal transition occurs, vascular endothelial cells lose their unique cobblestone-like shape and change into long and spindle-shaped mesenchymal cells. Through phalloidin staining that binds to F-actin, it was confirmed that changes in cell shape and cytoskeleton occurred in pulmonary artery endothelial cells (PAECs) that had undergone endothelial-mesenchymal transition due to the simultaneous treatment of TGF-β2 and IL-1β (FIG. 1C). As a result of comparing the degree of endothelial-mesenchymal transition when TGF-β2 and IL-1β were simultaneously treated for 1, 3, 5, and 7 days, protein expression changes of the mesenchymal cell markers except SNAIL were increased over time (FIG. 1D). The mRNA expression of the mesenchymal cell markers was also increased over time, but CDH2 and SNAIL were highest in the 1-day treatment group and then tended to gradually decrease (FIG. 1E). The expression of both mRNA and protein of the vascular endothelial cell markers was gradually decreased over time (FIGS. 1D and 1E). The immunocytochemical staining results showed the same results as the protein and RNA results under conditions of inducing endothelial-mesenchymal transition by the simultaneous treatment with TGF-β2 and IL-1β (FIG. 1F).

### <Example 2> Methyltransferase SET7/9, Which is Important Epigenetic Factor in Endothelial-Mesenchymal Transition Induction Process

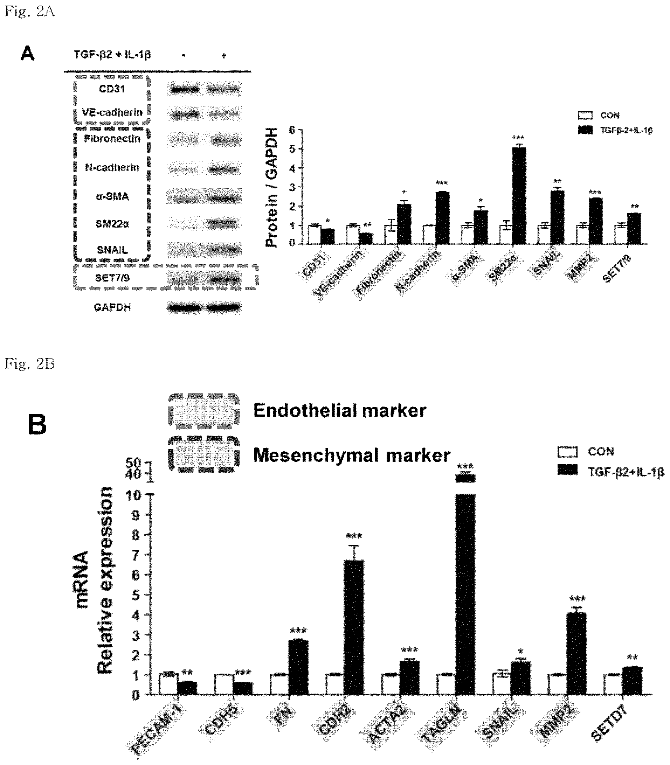

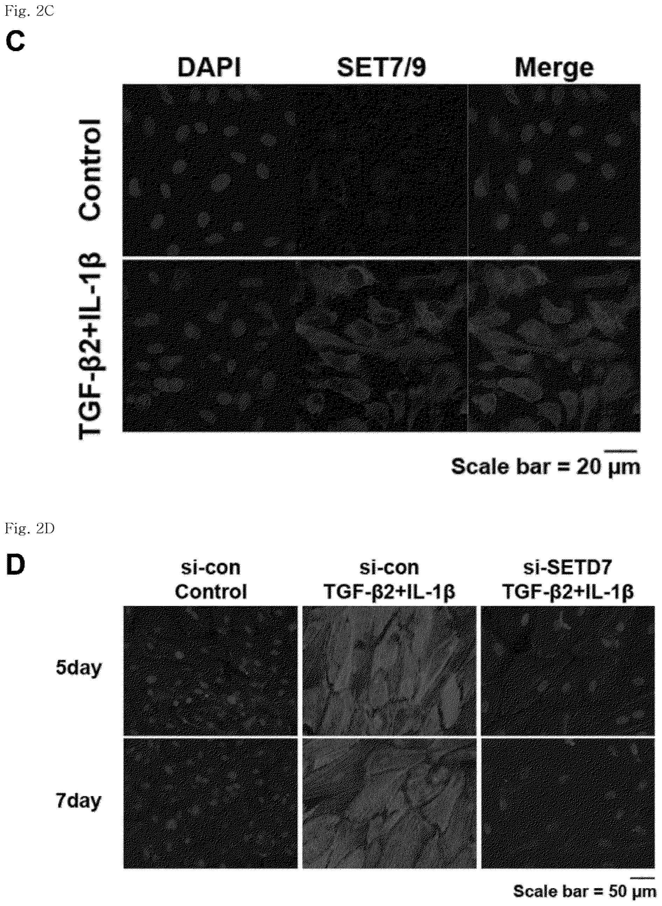

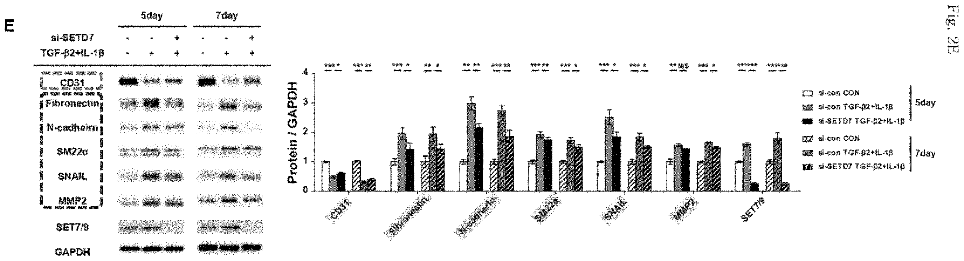

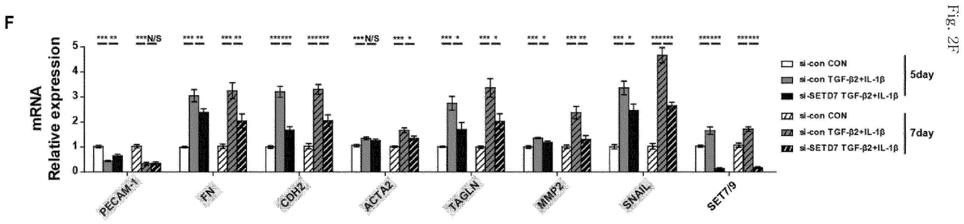

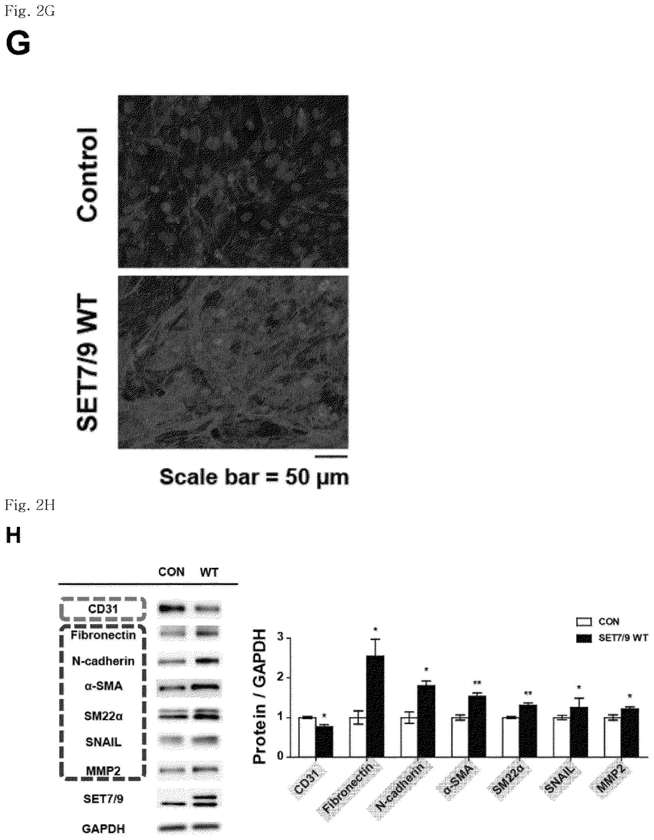

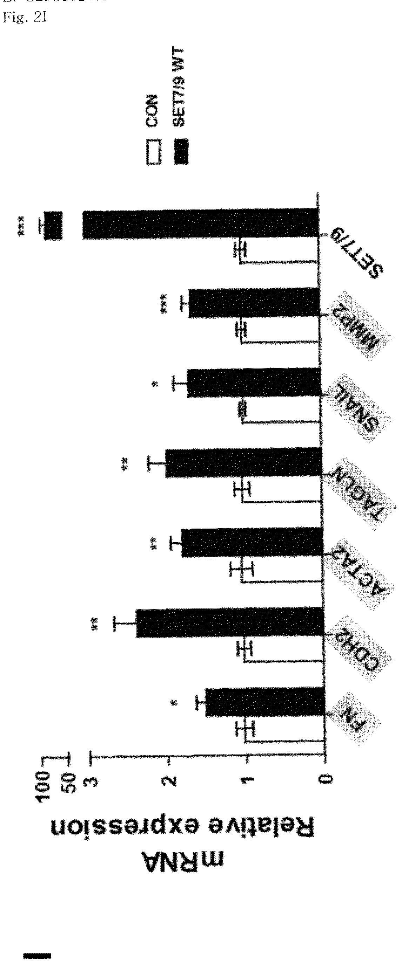

In order to find epigenetic factors that change significantly under endothelial-mesenchymal transition conditions induced by TGF-β2 and IL-1β, RT2 Profiler PCR Arrays were used. As a result of analysis using a scatter plot graph and a heat map, SET7/9 was discovered to be a strong candidate factor. As a result of Western blot (FIG. 2A), qRT-PCR (FIG. 2B), and immunocytochemical staining (FIG. 2C) experiments, it was confirmed that the protein and mRNA expression of SET7/9 was increased in pulmonary artery endothelial cells in which endothelial-mesenchymal transition was induced by the simultaneous treatment with TGF-β2 and IL-1β, which was consistent with the results of the epigenetic factor array. Next, the effect of SET7/9 knockdown was observed to investigate the relationship between endothelial-mesenchymal transition and SET7/9. It was confirmed by phalloidin staining that the degree of cell phenotypic change due to TGF-β2 and IL-1β-induced endothelial-mesenchymal transition was reduced upon SET7/9 knockdown (FIG. 2D). In addition, restoration of protein and mRNA expression of endothelial-mesenchymal transition markers by SET7/9 knockdown was confirmed by Western blot (FIG. 2E) and qRT-PCR (FIG. 2F). It was confirmed that, when SET7/9 was overexpressed, the shape of vascular endothelial cells changed to resemble mesenchymal cells (FIG. 2G), and protein and mRNA expression of the endothelial-mesenchymal transition markers also changed (FIGS. 2H and 2I).

### <Example 3> Inhibition of SET7/9, Which Regulates Pro-fibrotic Signaling and Inhibits Endothelial-Mesenchymal Transition

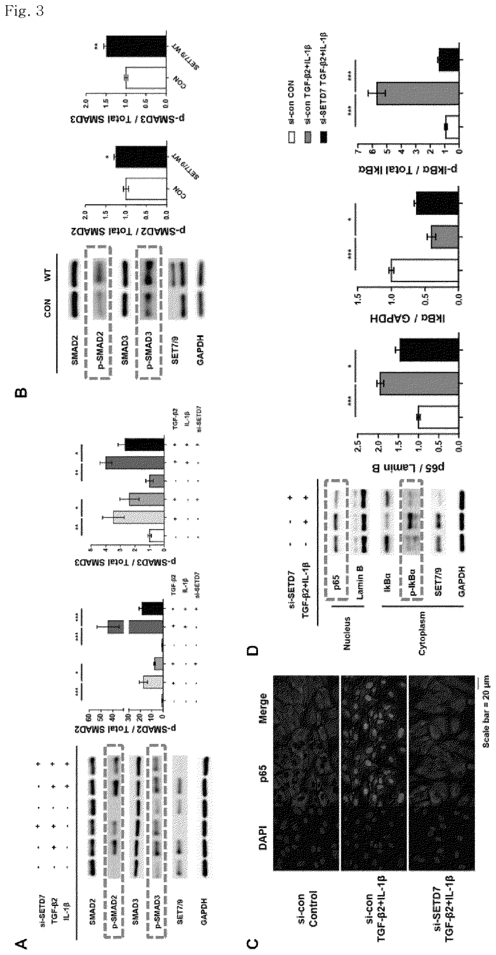

In order to study the downstream signaling system of endothelial-mesenchymal transition, studies on TGF-β-SMAD2/3 and NF-κB signaling, which are representative pro-fibrotic signaling, were conducted. It was confirmed whether SET7/9 regulates SMAD2/3 signaling by a treatment of TGF-β2 alone and a simultaneous treatment of TGF-β2 and IL-1β. As a result, it was confirmed that phosphorylated SMAD2/3 increased more in the group subjected to the simultaneous treatment of TGF-β2 and IL-1β than in the group subjected to the treatment of TGF-β2 alone, and the signaling was suppressed by SET7/9 knockdown (FIG. 3A). In addition, it was confirmed that phosphorylation of SMAD2/3 was increased by overexpression of SET7/9 (FIG. 3B). Next, it was confirmed by immunocytochemical staining that the expression of p65, which was increased during the simultaneous treatment of TGF-β2 and IL-1β, was decreased by SET7/9 knockdown (FIG. 3C). As a result of fractionation into cytoplasm and nucleus, it was confirmed that, due to the simultaneous treatment of TGF-β2 and IL-1β, the expression of phosphorylated IκBα was increased in the cytoplasm and NF-κB was accumulated in the nucleus, which were all restored by SET7/9 knockdown (FIG. 3D).

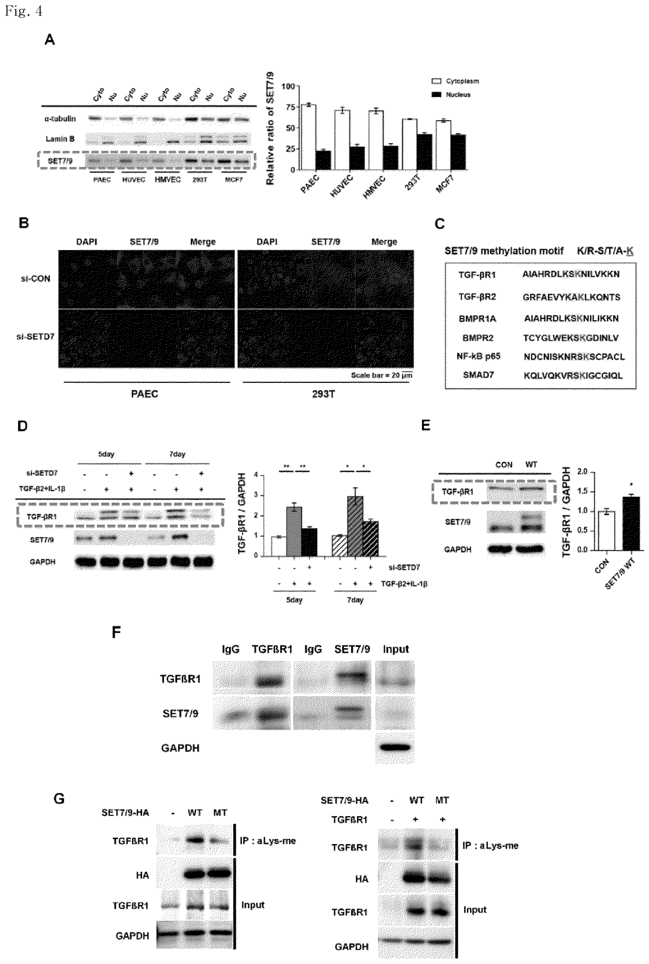

Because the results of the studies showed that SET7/9 was mainly located in the cytoplasm rather than the nucleus, the expression of SET7/9 was confirmed by fractionating five cells, PAEC, HUVEC, HMVEC, 293T, and MCF7, into the nucleus and cytoplasm. As a result, compared to 293T and MCF7, vascular endothelial cells showed higher expression in the cytoplasm than in the nucleus (FIGS. 4A and 4B). It was confirmed whether the K/R-S/T/A-K motif recognized and methylated by SET7/9 is present in proteins related to canonical TGF-β-SMAD2/3 and NF-κB signaling (FIG. 4C). Among them, the expression of TGF-βR1 was increased upon induction of endothelial-mesenchymal transition and was regulated by SET7/9 knockdown (FIG. 4D). In addition, it was confirmed that the protein expression of TGF-βR1 was increased by overexpression of SET7/9 (FIG. 4E). In order to reveal that TGF-βR1 is the target protein of SET7/9, immunoprecipitation was performed, and first, it was confirmed that SET7/9 and TGF-βR1 were bound at the endogenous level (FIG. 4F). In order to confirm that TGF-βR1 is methylated by SET7/9, SET7/9 wild type (WT) and mutant type (MT) in which amino acids important for methylation were mutated were overexpressed and immunoprecipitated with a methylation antibody, respectively. As a result, it was confirmed that methylation of TGF-βR1 was significantly decreased in the group that overexpressed SET7/9 mutant type (MT) (FIG. 4G).

### <Example 4> Inhibition of SET7/9 for Restoring Vascular Endothelial Cell Dysfunction Caused by Endothelial-Mesenchymal Transition

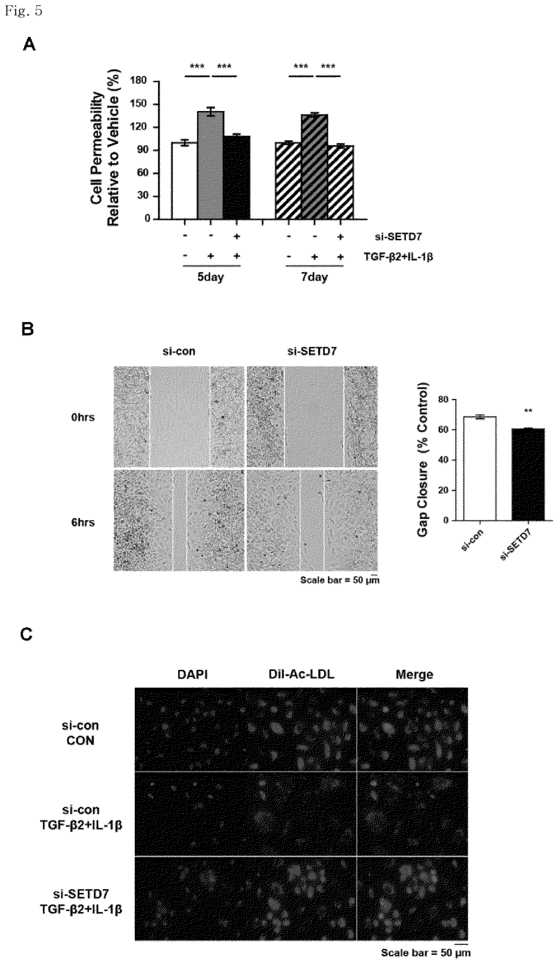

In order to confirm the role of SET7/9 in the vascular endothelial cell dysfunction, permeability, migration, and Dil-Ac-LDL uptake experiments were performed. It was confirmed that a degree of permeability increased by the induction of endothelial-mesenchymal transition was restored by SET7/9 knockdown (FIG. 5A). In addition, the migration of vascular endothelial cells was deceased by SET7/9 knockdown (FIG. 5B). The ability of vascular endothelial cells to uptake lipoproteins was decreased when endothelial-mesenchymal transition was induced, and it was confirmed that the decreased ability was restored by SET7/9 knockdown (FIG. 5C).

### <Example 5> Endothelial-Mesenchymal Transition Closely Related to Pulmonary Vascular Disease

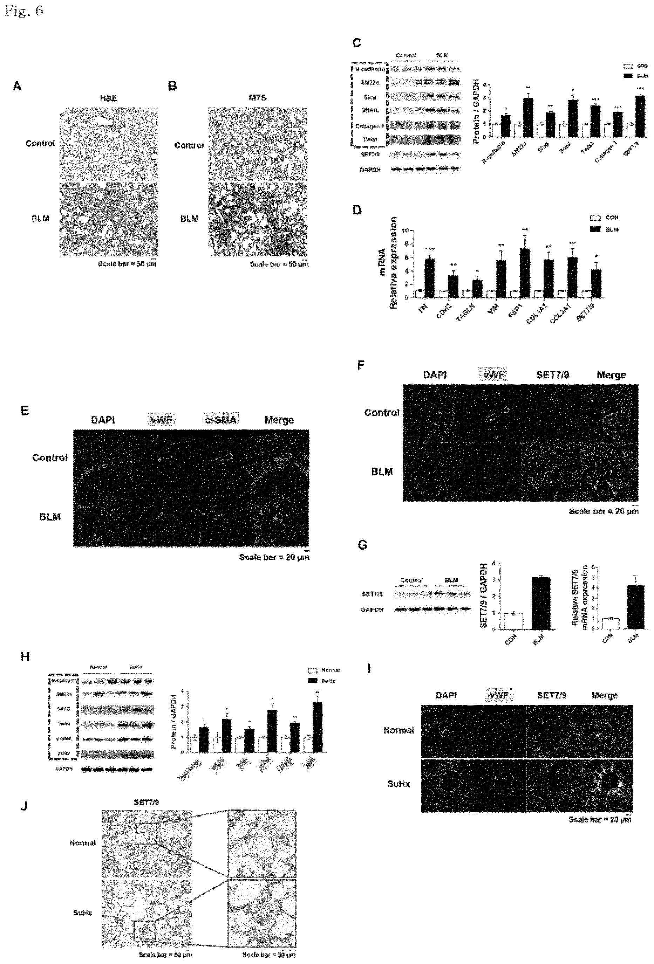

In order to confirm the relationship between endothelial-mesenchymal transition and pulmonary vascular disease, animal models with pulmonary fibrosis and pulmonary arterial hypertension were used. In the lung tissue of the bleomycin-induced pulmonary fibrosis mouse model, an increase in collagen and connective tissue was confirmed through hematoxylin eosin staining (H&E) and Masson's trichrome staining (MTS) (FIGS. 6A and 6B). Protein and mRNA expression of mesenchymal cell markers was increased in the lung tissue of the bleomycin-induced mice (FIGS. 6C and 6D). Using immunohistochemical staining, it was confirmed that, in the bleomycin group, the area where endothelial-mesenchymal transition in which the vascular endothelial cell marker vWF and the mesenchymal cell marker α-SMA were simultaneously stained occurred was increased (FIG. 6E). In addition, in the bleomycin group, an increase in protein and mRNA expression of SET7/9 was confirmed by immunofluorescence staining (FIG. 6F) and Western blot and qRT-PCR (FIG. 6G).

The Sugen/hypoxia (SuHx) rat model was used as an animal model with pulmonary arterial hypertension, and it was confirmed by Western blot that mesenchymal cell markers were significantly increased in the lung tissue of the SuHx group (FIG. 6H). In addition, an increase in expression of SET7/9 in blood vessels was confirmed using two immunohistochemical staining methods (FIGS. 6I and 6J).

### <Example 6> Restoration of Endothelial-Mesenchymal Transition by SET7/9 Inhibitor Cyproheptadine

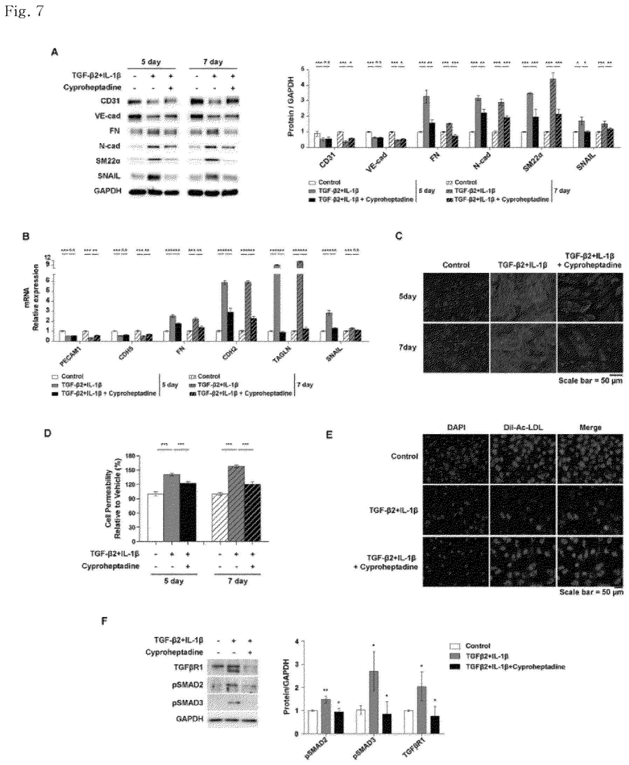

Cyproheptadine is a drug that has been reported to inhibit SET7/9. In order to confirm the inhibitory effect of the drug on endothelial-mesenchymal transition, a model in which pulmonary artery endothelial cells were simultaneously treated with cyproheptadine along with TGF-β2 and IL-1β for 5 to 7 days was used. As a result, restoration of the expression of endothelial cell markers CD31 (PECAM1) and VE-cadherin (CDH5) and mesenchymal cell markers Fibronectin (FN), N-cadherin (CDH2), SM22α (TAGLN), and SNAIL was confirmed by Western blot (FIG. 7A) and qRT-PCR (FIG. 7B). In the Cyproheptadine-treated group, it was confirmed through phalloidin staining that the cytoskeleton change, a characteristic of endothelial-mesenchymal transition, was decreased (FIG. 7C). It was confirmed that an increase in permeability (FIG. 7D) and a decrease in Dil-Ac-LDL uptake ability (FIG. 7E), which are the endothelial dysfunction caused by endothelial-mesenchymal transition, were restored by cyproheptadine. At this time, it was confirmed that the expression of TGFβR1, which was increased due to the induction of endothelial-mesenchymal transition by cyproheptadine, was restored and the degree of phosphorylation of SMAD2/3, a downstream signal, was decreased (FIG. 7F).

### <Example 7> Effect of SET7/9 Inhibitor Cyproheptadine in SuHx-Induced Pulmonary Arterial Hypertension Animal Model

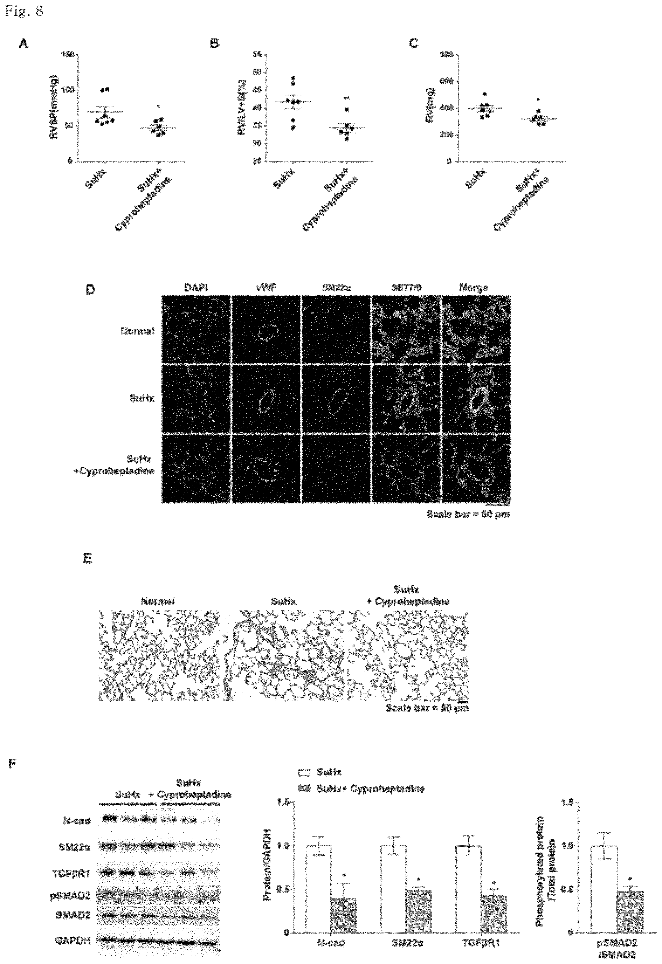

When cyproheptadine was administered intraperitoneally to SuHx-induced pulmonary arterial hypertension rats at a concentration of 5 mg/kg for 2 weeks, it was confirmed that the right ventricular systolic pressure (RVSP mmHg), the right ventricle/left ventricle + septum ratio (RV/LV+S %), and the right ventricular weight (RV mg) were decreased (FIGS. 8A to 8C). As a result of immunohistochemical staining, it was confirmed that muscularization was observed in blood vessels with a size of approximately 50 um in SuHx rat lung tissue, and a degree of muscularization was decreased when cyproheptadine was administered. In SuHx lung tissue, the phenomenon of endothelial-mesenchymal transition, in which endothelial and mesenchymal cell markers were simultaneously stained, was increased, and it was observed that the expression of SET7/9 was increased in blood vessels where this phenomenon occurred. However, in the cyproheptadine administration group, it was confirmed that endothelial-mesenchymal transition was inhibited through a decrease in SET7/9 expression in blood vessels and a decrease in area where simultaneous staining of endothelial and mesenchymal cell markers (FIG. 8D). Through H&E staining, it was confirmed that the number of blocked or enlarged blood vessels in the lung tissue of the rat group to which cyproheptadine was administered was reduced compared to the SuHx-induced pulmonary arterial hypertension group (FIG. 8E). In the lung tissue of the rat group to which cyproheptadine was administered, it was confirmed that the expression of mesenchymal cell markers such as N-cad and SM22α and TGFβR1, a target protein of SET7/9, was decreased, and the expression of phosphorylated SMAD2 protein was significantly decreased (FIG. 8F).

### <Example 8> Restoration of Endothelial-Mesenchymal Transition by SET7/9 Specific Inhibitor (R)-PFI-2

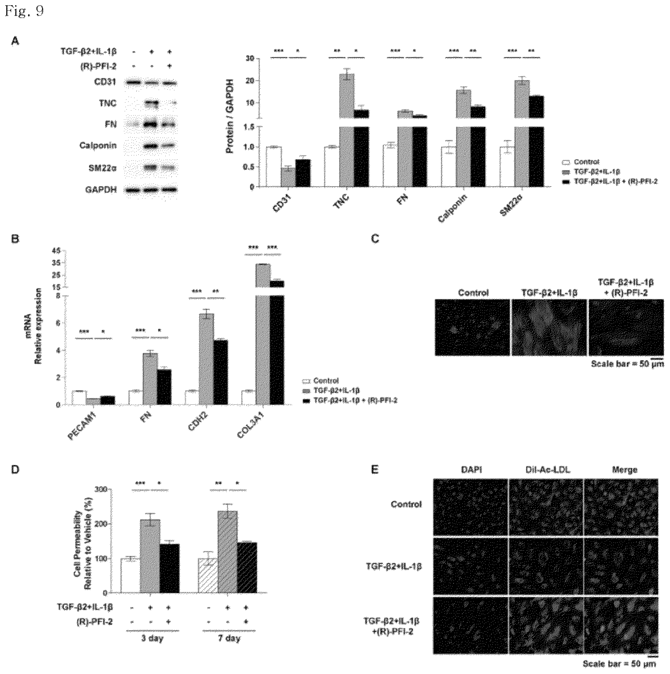

(R)-PFI-2 is a substrate competitive inhibitor that acts by attaching to the substrate peptide binding groove of SET7/9. Therefore, in order to confirm the effect of restoring endothelial-mesenchymal transition by (R)-PFI-2, a SET7/9 specific inhibitor, a model in which pulmonary artery endothelial cells were simultaneously treated with TGF-β2, IL-1β, and (R)-PFI-2 was used. As a result, restoration of the expression of the endothelial cell marker CD31 and the mesenchymal cell markers TNC, FN, Calponin, and SM22α was confirmed by Western blot (FIG. 9A), and restoration of the expression of the endothelial cell marker PECAM1 and the mesenchymal cell markers FN, CDH2, and COL3A1 was confirmed by qRT-PCR (FIG. 9B). Through phalloidin staining, it was confirmed that the change in cytoskeleton caused by (R)-PFI-2 was reduced (FIG. 9C) and the permeability (FIG. 9D) and the Dil-Ac-LDL uptake ability (FIG. 9E) were restored.

### <Example 9> Effect of SET7/9 Specific Inhibitor (R)-PFI-2 in SuHx-Induced Pulmonary Arterial Hypertension Animal Model

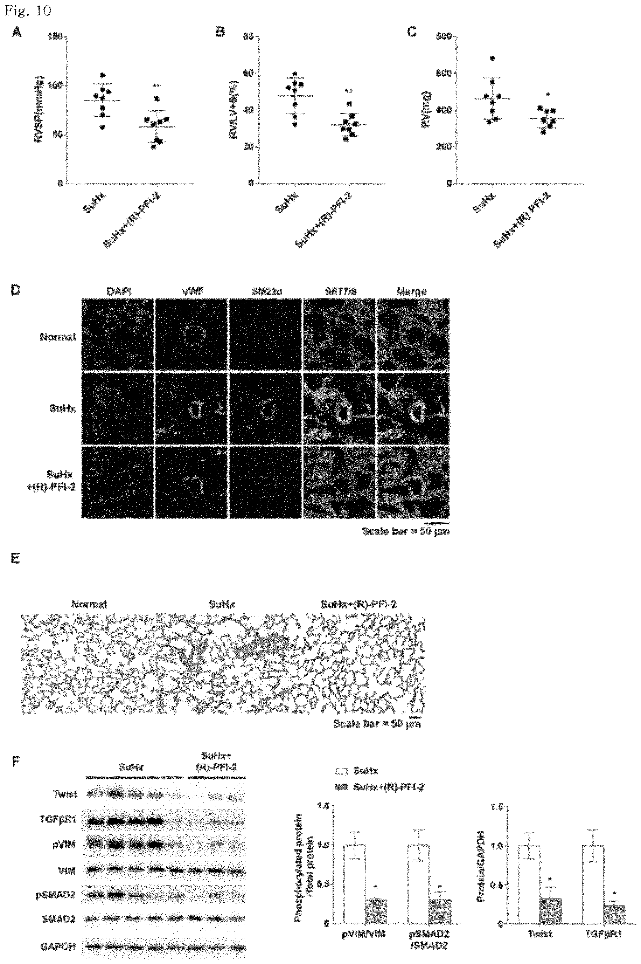

In order to confirm the effect of (R)-PFI-2 in an animal model, (R)-PFI-2 was administered subcutaneously to SuHx-induced pulmonary arterial hypertension rats at a concentration of 10 mg/kg for 3 weeks. In the (R)-PFI-2 administration group, it was confirmed that the right ventricular systolic pressure (RVSP mmHg), the right ventricle/left ventricle + septum ratio (RV/LV+S %), and the right ventricle weight (RV mg) were decreased (FIGS. 10A to 10C). As a result of immunohistochemical staining, the muscularization of SuHx rat lung tissue blood vessels was significantly reduced when (R)-PFI-2 was administered. In addition, it was observed that the expression of SET7/9 was high in blood vessels where endothelial-mesenchymal transition occurred in SuHx lung tissue, and in the (R)-PFI-2 administration group, it was confirmed that endothelial-mesenchymal transition was inhibited through a decrease in SET7/9 expression in blood vessels and a decrease in area where endothelial and mesenchymal cell markers were simultaneously stained (FIG. 10D). Through H&E staining, it was confirmed that the number of blocked or enlarged blood vessels in the lung tissue of the rat group to which (R)-PFI-2 was administered was reduced compared to the SuHx-induced pulmonary arterial hypertension group (FIG. 10E). In the lung tissue of the rat group to which (R)-PFI-2 was administered, it was confirmed that the expression of mesenchymal cell markers such as Twist and pVIM and TGFβR1, a target protein of SET7/9, was decreased, and the expression of phosphorylated SMAD2 protein was significantly decreased (FIG. 10F).

Special portions of the present invention have been described in detail hereinabove, and it will be obvious to those skilled in the art that this detailed description is only an exemplary embodiment and the scope of the present invention is not limited by this detailed description. Therefore, the substantial scope of the present invention will be defined by the claims and equivalents thereof.

## Claims

1. A biomarker composition for diagnosing an endothelial-mesenchymal transition (EndMT)-related disease, the biomarker composition comprising methyltransferase SET7/9 protein or a gene encoding methyltransferase SET7/9 protein as an active ingredient.

2. The biomarker composition of claim 1, wherein the EndMT-related disease is pulmonary fibrosis or pulmonary arterial hypertension.

3. A composition for diagnosing an EndMT-related disease, the composition comprising an agent capable of measuring an expression level of methyltransferase SET7/9 as an active ingredient.

4. The composition of claim 3, wherein the agent capable of measuring an expression level of SET7/9 is a primer or probe that specifically binds to the SET7/9 gene, or an antibody, peptide, aptamer, or compound that specifically binds to the SET7/9 protein.

5. The composition of claim 3, wherein the EndMT-related disease is pulmonary fibrosis or pulmonary arterial hypertension.

6. A kit for diagnosing an EndMT-related disease, the kit comprising the composition of any one of claims 3 to 5.

7. A method for providing information required for diagnosing an EndMT-related disease, the method comprising:
(1) measuring an mRNA expression level of SET7/9 gene or an expression level of SET7/9 protein from a sample isolated from a patient;
(2) comparing the mRNA expression level of the SET7/9 gene or the expression level of the SET7/9 protein with an expression level of a control sample; and
(3) determining that a disease is an EndMT-related disease when the mRNA expression level of the SET7/9 gene or the expression level of the SET7/9 protein is higher than that of the control sample.

8. The method of claim 7, wherein the EndMT-related disease is pulmonary fibrosis or pulmonary arterial hypertension.

9. A method for screening a therapeutic agent for an EndMT-related disease, the method comprising:
(1) bringing test substances into contact with vascular endothelial cells;
(2) measuring an expression or activity level of SET7/9 protein in the vascular endothelial cells in contact with the test substances; and
(3) screening a test substance having a reduced expression or activity level of the SET7/9 protein compared to a control sample.

10. The method of claim 9, wherein the EndMT-related disease is pulmonary fibrosis or pulmonary arterial hypertension.

11. A pharmaceutical composition for preventing or treating pulmonary arterial hypertension, the pharmaceutical composition comprising a SET7/9 expression or activity inhibitor as an active ingredient.

12. The pharmaceutical composition of claim 11, wherein the SET7/9 expression or activity inhibitor is (R)-PFI-2.

13. A reagent composition for inhibiting EndMT in pulmonary artery endothelial cells in vitro or in pulmonary arterial hypertension rat models in vivo, the reagent composition comprising a SET7/9 expression or activity inhibitor as an active ingredient.

14. The reagent composition of claim 13, wherein the SET7/9 expression or activity inhibitor is cyproheptadine or (R)-PFI-2.

15. A method for inhibiting EndMT in pulmonary artery endothelial cells or in pulmonary arterial hypertension rat models, the method comprising treating pulmonary artery endothelial cells in vitro or pulmonary arterial hypertension rat models in vivo with a SET7/9 expression or activity inhibitor.

16. The method of claim 15, wherein the SET7/9 expression or activity inhibitor is cyproheptadine or (R)-PFI-2.
